# EUROPEAN PATENT SPECIFICATION

(11) **EP 0 535 129 B1**
(45) Date of publication and mention of the grant of the patent: **15.03.1995**
(21) Application number: 91912208.5
(22) Date of filing: 30.05.1991
(51) Int. Cl.: A61K 38/55, A61K 31/725

(54) **AN ANTICOAGULANT PREPARATION**
ANTIKOAGULANSZUBEREITUNG
PREPARATION ANTICOAGULANTE

(30) Priority: 19.06.1990 DK 1488/90
(43) Date of publication of application: 07.04.1993
(73) Proprietor: NOVO NORDISK A/S, 2880 Bagsvaerd (DK)
(72) Inventor: NORDFANG, Ole, Juul, DK-3400 Hilleroed (DK)
(86) International application number: DK9100145
(87) International publication number: WO9119514

(56) References cited:
- WO-A-91/02753
- US-A- 4 900 723
- Chemical Abstracts, volume 109, no. 13, 26 September 1988,(Columbus, Ohio, US), Sandset, Per Morten et al.:"Heparin induces release of extrinsic coagulation pathwayinhibitor (EPI).", see page 43, abstract no. 104418c, & Thromb. Res. 1988, 50(6), 803-813
- Sandset et al 1988 Trombusis Res. 50:803-813 (Full version)

## Description

### FIELD OF INVENTION

The present invention relates to a preparation for treating coagulation disorders or cancer, which preparation comprises a protein with anticoagulant activity and a substance acting synergistically with said protein. The invention further relates to a method of treating coagulation disorders or cancer by means of this preparation.

### BACKGROUND OF THE INVENTION

Blood coagulation is a complex process involving many activating and inactivating coagulation factors. Anticoagulant proteins are known to be important for regulation of the coagulation process (see B. Lämmle and J. Griffin (Clinics in Haematology 14 (1985), 281-342) and anticoagulants are thus important in the treatment of a variety of diseases, eg thrombosis, myocardial infarction, disseminated intravascular coagulation etc.

Thus heparin is used clinically to increase the activity of antithrombin III and heparin cofactor II. Antithrombin III is used for the inhibition of factor Xa and thrombin. Hirudin is used for the inhibition of thrombin and protein C may be used for the inhibition of factor V and VIII.

Anticoagulant proteins may also be used in the treatment of cancer. Thus, antistatin has been shown to have antimetastatic properties (J.H. Han et al., Gene 75 (1989), (47-57). Also heparin and warfarin have been shown to possess antimetastatic properties (G.J. Gasic et al., Int. Rev. Exp. Pathol. 29 (1985), 173-209).

Coagulation can be initiated through the extrinsic pathway by the exposure of tissue factor (TF) to the circulating blood (Y. Nemerson, Blood 71 (1988), 1-8). Tissue factor is a protein cofactor for FVII/VIIa and binding of tissue factor enhances the enzymatic activity of FVIIa towards its substrates FIX and FX. Placenta anticoagulant protein is able to inhibit tissue factor activity, probably by interfering with TF/FVIIa-phospholipid interaction (S. Kondo et al., Thromb. Res. 48 (1987), 449-459).

Recently a new anticoagulant protein, the extrinsic pathway inhibitor (EPI) has been isolated (Broze et al., Proc. Natl. Acad. Sci. 84 (1987), 1886-1890).

On a molar basis EPI has been shown to be a far more potent inhibitor of TF/FVIIa induced coagulation than the placenta anticoagulant protein (R.A. Gramzinski et al., Blood 73 (1989), 983-989). EPI binds and inhibits FXa and the complex between EPI and Xa inhibits TF/FVIIa (SI Rapaport, Blood 73 (1989), 359-365). EPI is especially interesting as an anticoagulant/antimetastatic agent as many tumor cells express TF activity (T. Sakai et al., J. Biol. Chem. 264 (1989), 9980-9988) and because EPI shows anti-Xa activity like antistatin.

EPI has been recovered by Broze et al. (supra) from HepG2 hepatoma cells (Broze, DK patent application No. 4135/88). The gene for the protein has been cloned and the protein has been shown to consist of 3 tandem Kunitz type inhibitor domains (Broze, DK patent application No. 3907/88). The protein consists of 276 amino acid residues and has in addition to the three Kunitz type inhibitor domains three potential glycosylation sites at position Asn117, Asn167 and Asn229. The molecular weight indicates that some of these sites are glycosylated. Furthermore, it has been shown that Kunitz domain 2 binds FXa while the first Kunitz domain binds FVIIa/TF (Girard et al., Nature 338 (1989), 518-520). EPI has also been isolated from Hela cells (DK
patent application No. 6199/88) and it was shown that HeLa EPI binds heparin.

### DISCLOSURE OF THE INVENTION

The present invention relies on the possibility of increasing the anticoagulant activity and the half-life of injected EPI in plasma (i.e. the period of time when EPI circulates in the blood vessels) by concomitantly administering heparin.

Accordingly, the present invention relates to a pharmaceutical preparation for the prophylaxis or treatment of coagulation disorders or cancer, which comprises an extrinsic pathway inhibitor (EPI) protein and heparin or another mucopolysaccharide together with a pharmaceutically acceptable diluent or vehicle.

Thus, the present invention utilises the finding that EPI is a heparin-binding protein (cf. Danish Patent Application No. 6199/88). Furthermore, studies by Sandset et al. (Thromb. Res. 50, 1988, pp. 803-813) have shown that the natural plasma level of EPI is increased up to two-fold following the subcutaneous injection of heparin. These findings have led the present inventors to assume that circulating heparin prevents the binding of EPI to heparin-like substances (primarily mucopolysaccharides) on endothelial cell surfaces. Conversely, heparin may also act to release already bound EPI from the endothelium. In any case, it is believed to be essential for the anticoagulant activity of EPI that it is found in the circulation rather than bound to endothelial surfaces. Heparin may therefore be said to exert a synergistic effect on the activity of EPI in that, by binding free EPI or EPI released from the endothelium, it increases the anticoagulant activity of EPI and enables the EPI to circulate in the blood. In vitro studies have shown that heparin increases the activity of antithrombin III (L. Rosenfeld, Biochem. J. 237, 1986, pp. 639-646). However, no increase in the half-life of injected antithrombin III on injection of heparin has been observed, and heparin does not act synergistically with antithrombin III in the case of disseminated intravascular coagulation (cf. B. Blauhut, Thromb. Res. 39, 1985, pp. 81-89).

It should be noted that the present invention provides as well preparations in which the EPI protein is combined with heparin in such a way that the EPI protein is actually bound to heparin before administration, as preparations in which the EPI protein and the heparin are kept in separate containers before use in a form which is adapted to the substantially simultaneous or sequential co-administration of the EPI protein and heparin (e.g. with a content of EPI protein adapted to the intended use of the preparation, and with a content of heparin which is sufficient to bind substantially all the EPI protein to be administered). In the latter case, the EPI protein will be bound to circulating heparin in the blood vessels upon administration of both substances.

The coagulation disorders which are to be treated by means of the preparation of the invention are primarily disorders which require treatment with an anticoagulant. Examples of such disorders are those which are conventionally treated by administering heparin alone, e.g. thombosis, embolism, infarctions or disseminated intravascular coagulation. The preparation of the invention is also contemplated to be useful in the treatment of cancer. This utility is suggested by the anti-metastatic properties of other anticoagulants such as antistatin (cf. J.H. Han et al., Gene 75, 1989, pp. 47-57), heparin and warfarin (cf. G.J. Gasic et al., Int. Rev. Exp. Pathol. 29, 1985, pp. 173-209).

In the present context, the term "EPI protein" is intended to include not only native, or full-length, EPI but also EPI analogues with affinity for heparin. Examples of such analogues are EPI fragments which include the heparin binding domain (believed to be located within the region of the native EPI molecule from the amino acid residue in position 165 to the C-terminal amino residue in position 276, and more specifically assumed to comprise a region rich in positively charged amino acid residues from Arg246 to Lys265).

The term "another mucopolysaccharide" is intended to include heparin-like substances with the ability to bind EPI. Examples of such substances are sulfated glucosaminoglycans selected from heparan sulfate, dermatan sulfate and protamine sulfate. However, the currently preferred mucopolusaccharide for the present purpose is heparin, and the invention is explained herein mainly in terms of heparin although this should not be construed as a limitation of the invention to the use of heparin.

The preparation of the invention may be compounded in any form which is suitable for parenteral administration (e.g. for intravenous or subcutaneous injection or infusion), for instance by dissolving or suspending the EPI protein and the heparin, either separately or in admixture, as explained above, in sterile water or isotonic saline. The dosage level needed to achieve the desired therapeutic effect is estimated on the basis of the content of native EPI in the blood vessels of healthy individuals and the amount of heparin needed to release it from the endothelium. EPI is present in the blood of healthy individuals in an amount of 50 ng/ml of plasma. Injection of, e.g., 5000 IU of heparin may in theory give rise to the release of EPI from epithelial surfaces to a concentration of up to 500ng/ml of plasma. In order to obtain a significant anticoagulant effect of the EPI protein, it is contemplated that a suitable dosage of EPI (unit dose) may be in the range of about 0.5 -40 mg EPI, i.a. dependent on the type and severity of the condition for which treatment with EPI is indicated. A corresponding suitable dosage of heparin is one which is capable of binding this amount of EPI protein to keep it in circulation. Thus, the dosage of heparin to be co-administered with the EPI protein may be in the range of about 1000-15000 IU per unit dose, such as in the range of 2000-10000 IU per unit dose, in particular about 5000 IU per unit dose.

In another aspect, the present invention relates to a method of treating or preventing coagulation disorders or cancer, which comprises administering, to a patient in need of such treatment, a therapeutically or prophylactically effective dosage of EPI and heparin.

In one embodiment of the present method, the administration of the EPI protein may be substantially simultaneous with the administration of heparin. This may, for instance be effected by mixing the EPI protein with heparin prior to administration so that the EPI protein will be administered in a form in which it is bound to heparin, or the EPI protein and heparin may be administered separately by means of a device which makes it possible to administer two substances simultaneously. Finally, either the EPI protein or heparin may be administered first and the other component may be administered immediately after that ("immediately" meaning any period of time up to one minute after administering the first substance).

In another embodiment, the EPI protein may be administered before the administration of heparin. In this case, it is expected that the EPI protein will circulate for only a brief period of time (typically ten minutes) after which it will be bound to heparin-like mucopolysaccharides on epithelial cell surfaces and thus be inactivated. It is, however, envisaged that, analogously with another blood protein (platelet factor 4; cf. G. Cella et al., Eur. J. Clin. Invest. 17, 1987, pp. 548-554), the bound EPI will be released by the subsequent administration of heparin and bind to the administered circulating heparin instead.

In an alternative embodiment, the EPI protein may be administered after the administration of heparin. In this case, the EPI protein will be bound to the circulating heparin substantially immediately after administration. In order to obtain the desired synergistic effect of heparin, it is contemplated that the EPI protein may be administered 0-24, preferably 0-2, and most preferably 0-0.5, hours after the administration of heparin.

In the latter two embodiments, the EPI protein and the heparin will be administered separately from separate containers. As indicated above, the EPI may be administered in an amount of 0.5 - 40 mg EPI, and the heparin may be administered in an amount of 1000-15000 IU per unit dose, such as an amount of 2000-10000 IU per unit dose. The coagulation disorders for which the administration of EPI protein and heparin is indicated may be any of those mentioned above.

In a further aspect, the present invention relates to the use of an EPI protein and heparin for preparing a medicament for the prophylaxis or treatment of coagulation disorders or cancer. As discussed in more detail above, the EPI protein may be bound to heparin prior to administration, or the EPI protein and the heparin may be provided in separate containers in a form adapted to the substantially simultaneous or sequential co-administration of EPI protein and heparin.

The following method may be employed to show EPI activity in plasma after administration of the present preparation.

Assay for EPI activity: EPI was measured in a chromogenic microplate assay, modified after the method of Sandset et al., (Thromb. Res. 47 (1989), 389-400). Heat treated plasma pool was used as a standard. This standard is set to contain 1 U/ml of EPI activity. Standards and samples were diluted in buffer A (0.05 M tris / 0.1 M NaCl / 0.1 M Na-citrate / 0.02% NaN₃ / pH 8.0) containing 2 ug/ml polybrene and 0.2% bovine serum albumin. FVIIa/TF/FX/CaCl₂ combination reagent was prepared in buffer A and contained 1.6 ng/ml FVIIa (Novo-Nordisk a/s), human tissue factor diluted 60 fold (Hjort, Scand. J. Clin. Lab. Invest. 9 (1957), 50 ng/ml FX (Sigma) and 18 mM CaCl₂. The assay was performed in microplate strips at 37°C. 50 ul of samples and standards were pipetted into the strips and 100 ul combination reagent was added to each well. After 10 minutes incubation, 25 ul of FX (3.2 ug/ml) was added to each well and after another 10 minutes 25 ul of chromogenic substrate for FXa (S2222) was added 10 minutes after the addition of substrate. The reaction was stopped by addition of 50 ul 1.0 M citric acid pH 3.0. The microplate was read at 405 nm.

### Coagulation assays

APTT assay: In the Activated Partial Thromboplastin Time (APTT) assay, 55 »l of plasma incubation mixture was mixed with 55 »l of APTT reagent for 300 seconds at 37° C before 55 »l of 0.025 M CaCl₂ were added, and the coagulation time was measured.

PT assay: In the Prothrombin Time (PT) assay rabbit thromboplastin was dissolved according to the manufacturers instructions and 1 volume of thromboplastin was mixed with 2 volumes of 0.03 M CaCl₂. In the assay 75 »l of incubation mixtures was mixed with 75 »l of thromboplastin/CaCl₂ reagent at 37° C before the coagulation time was measured.

Dilute tissue factor (dTF) assay: The dTF assay was similar to the PT assay. However, in this assay we used human thromboplastin diluted 7.000 fold in coagulation buffer as opposed to be undiluted rabbit thromboplastin used in the PT assay.

### EXAMPLE

### Demonstration of a synergistic effect in coagulation.

Coagulation assays were made on plasma samples with added EPI and/or LMW heparin, alle diluted in coagulation buffer (0.1% bovine serum albumin, 50 mM imidazole, 100 mM Nacl, pH 7.3). One sixth of the total volume was rEPI, 1/20 of the volume was LMW heparin. In samples where some of these reagents were not added, coagulation buffer was added to keep the dilution of plasma constant. All samples were incubated for 15 minutes at room temperature before starting the assay. All clotting times were measured on an ACL 300 R coagulation apparatus from Instumentation Laboratories, Ascoli Piceno, Italy.

### RESULTS

The results are shown in Figures 1-3.

From Fig. 1 (APTT assay) it can be seen that addition of 10 »g/ml of rEPI alone increased the APTT coagulation time of normal human plasma by 26 seconds while addition of LMW heparin (0.4 FXaI U/ml) alone increased the time by 57 seconds. Coincubation of the two components in amounts as mentioned above resulted in a much greater effect namely prolonging the coagulation time by 283 seconds.

Fig. 2 (PT assay) shows that addition of 4 »g/ml rEPI alone increased the coagulation time of normal plasma by 4.4 seconds while addition of LMW heparin (2 FXaI U/ml alone increased the time by 10.4 seconds. Coincubation of the two components in amounts as mentioned above prolonged the coagulation time by as much as 141 seconds.

Fig. 3 (dTF assay) shows that addition of 0.8 »g/ml of rEPI alone increased the cogulation time of normal plasme by 35 seconds while addition of LMW heparin (0.2 FXaI U/ml) alone increased the time by 105 seconds. Coincubation of the two components in amounts as mentioned above prolonged the coagulation time by 341 seconds.

Thus, in all three assays the results show a synergistic effect between rEPI and LMW heparin.

## Claims (Claims for the following Contracting State(s): AT, BE, CH, DE, DK, FR, GB, IT, LI, LU, NL, SE)

1. A pharmaceutical preparation for the prophylaxis or treatment of coagulation disorders or cancer, which comprises an extrinsic pathway inhibitor (EPI) protein and heparin or other mucopolysaccharide together with a pharmaceutically acceptable diluent or vehicle.

2. A preparation according to claim 1, wherein the EPI protein is bound to heparin.

3. A preparation according to claim 1, which comprises EPI protein and heparin in separate containers in a form which is adapted to the substantially simultaneous or sequential co-administration of EPI protein and heparin.

4. A preparation according to any of claims 1-3, wherein the EPI protein is present in an amount of about 0.5 - 40 mg.

5. A preparation according to any of claims 1-4, wherein the heparin is present in an amount of 1000-15000 IU per unit dose, such as an amount of 2000-10000 IU per unit dose.

6. Use of an EPI protein and heparin for preparing a medicament for the prophylaxis or treatment of coagulation disorders or cancer.

7. Use according to claim 6, wherein the EPI protein is bound to heparin.

8. Use according to claim 6, wherein the EPI protein and the heparin are provided in separate containers in a form adapted to the substantially simultaneous or sequential co-administration of EPI protein and heparin.

## Claims (Claims for the following Contracting State(s): ES, GR)

1. A method of preparing a pharmaceutical preparation comprising an extrinsic pathway inhibitor (EPI) protein and heparin or another mucopolysaccharide, wherein the EPI protein is dissolved or suspended in a pharmaceutically acceptable diluent or vehicle followed by admixture with the heparin or other mucopolysaccharide whereby the EPI protein is bound to the heparin or other mucopolysaccharide.

2. A method according to claim 1, wherein the EPI protein is added in an amount corresponding to about 0.5-40 mg per unit dose.

3. A method according to claim 1 or 2, wherein the heparin is added in an amount corresponding to 1000-15000 IU per unit dose, such as an amount corresponding to 2000-10000 IU per unit dose.

4. Use of an EPI protein and heparin for preparing a medicament for the prophylaxis or treatment of coagulation disorders or cancer.

5. Use according to claim 4, wherein the EPI protein is bound to heparin.

6. Use according to claim 4, wherein the EPI protein and the heparin are provided in separate containers in a form adapted to the substantially simultaneous or sequential co-administration of EPI protein and heparin.

## Patentansprüche (Patentansprüche für folgende(n) Vertragsstaat(en): AT, BE, CH, DE, DK, FR, GB, IT, LI, LU, NL, SE)

1. Eine pharmazeutische Zubereitung für die Prophylaxe oder Behandlung von Gerinnungsstörungen oder Krebs, die ein Extrinsic-Pathway-Inhibitor(EPI)-Protein und Heparin oder anderes Mucopolysaccharid zusammen mit einem pharmazeutisch annehmbaren Verdünnungsmittel oder Trägerstoff umfaßt.

2. Eine Zubereitung nach Anspruch 1, wobei das EPI-Protein an Heparin gebunden ist.

3. Eine Zubereitung nach Anspruch 1, die EPI-Protein und Heparin in getrennten Behältern in einer Form umfaßt, die an die im wesentlichen gleichzeitige oder aufeinanderfolgende Zusammenverabreichung von EPI-Protein und Heparin angepaßt ist.

4. Eine Zubereitung nach einem der Ansprüche 1-3, wobei das EPI-Protein in einer Menge von etwa 0,5-40 mg vorhanden ist.

5. Eine Zubereitung nach einem der Ansprüche 1-4, wobei das Heparin in einer Menge von 1.000-15.000 IU pro Einheitsdosis vorhanden ist, wie etwa in einer Menge von 2.000-10.000 IU pro Einheitsdosis.

6. Verwendung eines EPI-Proteins von Heparin zur Herstellung eines Arzneimittels für die Prophylaxe oder Behandlung von Gerinnungsstörungen oder Krebs.

7. Verwendung nach Anspruch 6, wobei das EPI-Protein an Heparin gebunden ist.

8. Verwendung nach Anspruch 6, wobei das EPI-Protein und das Heparin in getrennten Behältern in einer Form zur Verfügung gestellt werden, die an die im wesentlichen gleichzeitige oder aufeinanderfolgende Zusammenverabreichung von EPI-Protein und Heparin angepaßt ist.

## Patentansprüche (Patentansprüche für folgende(n) Vertragsstaat(en): ES, GR)

1. Ein Verfahren zur Herstellung einer pharmazeutischen Zubereitung, die ein Extrinsic-Pathway-Inhibitor(EPI)-Protein und Heparin oder ein anderes Mucopolysaccharid umfaßt, wobei das EPI-Protein in einem pharmazeutisch annehmbaren Verdünnungsmittel oder Trägerstoff gelöst oder suspendiert wird, gefolgt von der Vermischung mit dem Heparin oder anderen Mucopolysaccharid, wodurch das EPI-Protein an das Heparin oder anderes Mucopolysaccharid gebunden wird.

2. Ein Verfahren nach Anspruch 1, wobei das EPI-Protein in einer Menge zugegeben wird, die etwa 0,5-40 mg pro Einheitsdosis entspricht.

3. Ein Verfahren nach Anspruch 1 oder 2, wobei das Heparin in einer Menge zugegeben wird, die 1.000-15.000 IU Pro Einheitsdosis entspricht, wie etwa in einer Menge, die 2.000-10.000 IU pro Einheitsdosis entspricht.

4. Verwendung eines EPI-Proteins von Heparin zur Herstellung eines Arzneimittels für die Prophylaxe oder Behandlung von Gerinnungsstörung oder Krebs.

5. Verwendung nach Anspruch 4, wobei das EPI-Protein an Heparin gebunden ist.

6. Verwendung nach Anspruch 4, wobei das EPI-Protein und das Heparin in getrennten Behältern in einer Form zur Verfügung gestellt werden, die an die im wesentlichen gleichzeitige oder aufeinanderfolgende Zusammenverabreichung von EPI-Protein und Heparin angepaßt ist.

## Revendications (Revendications pour l'(les) Etat(s) contractant(s) suivant(s): AT, BE, CH, DE, DK, FR, GB, IT, LI, LU, NL, SE)

1. Préparation pharmaceutique pour la prophylaxie ou le traitement de troubles de la coagulation ou du cancer, qui comprend une protéine inhibitrice de la voie extrinsèque (EPI) et de l'héparine ou un autre mucopolysaccharide avec un diluant ou un véhicule pharmaceutiquement acceptable.

2. Préparation selon la revendication 1, dans laquelle la protéine EPI est liée à l'héparine.

3. Préparation selon la revendication 1, qui comprend la protéine EPI et l'héparine dans des récipients séparés sous une forme qui est adaptée à l'administration conjointe pratiquement simultanée ou successive de la protéine EPI et de l'héparine.

4. Préparation selon l'une quelconque des revendications 1-3, dans laquelle la protéine EPI est présente en une quantité d'environ 0,5 à 40 mg.

5. Préparation selon l'une quelconque des revendications 1-4, dans laquelle l'héparine est présente en une quantité de 1000 à 15000 UI par dose unitaire, par exemple en une quantité de 2000 à 10000 UI par dose unitaire.

6. Utilisation d'une protéine EPI et d'héparine pour la préparation d'un médicament pour la prophylaxie ou le traitement de troubles de la coagulation ou du cancer.

7. Utilisation selon la revendication 6, dans laquelle la protéine EPI est liée à l'héparine.

8. Utilisation selon la revendication 6, dans laquelle la protéine EPI et l'héparine sont présentées dans des récipients séparés sous une forme adaptée à l'administration conjointe pratiquement simultanée ou successive de la protéine EPI et de l'héparine.

## Revendications (Revendications pour l'(les) Etat(s) contractant(s) suivant(s): ES, GR)

1. Méthode de préparation d'une préparation pharmaceutique comprenant une protéine inhibitrice de la voie extrinsèque (EPI) et de l'héparine ou un autre mucopolysaccharide, dans laquelle on dissout ou on met en suspension la protéine EPI dans un diluant ou un véhicule pharmaceutiquement acceptable, puis on la mélange avec l'héparine ou l'autre mucopolysaccharide, grâce à quoi la protéine EPI est liée à l'héparine ou à l'autre mucopolysaccharide.

2. Méthode selon la revendication 1, dans laquelle la protéine EPI est ajoutée en une quantité correspondant à environ 0,5 - 40 mg par dose unitaire.

3. Méthode selon la revendication 1 ou 2, dans laquelle l'héparine est ajoutée en une quantité correspondant à 1000 à 15000 UI par dose unitaire, par exemple en une quantité de 2000 à 10000 UI par dose unitaire.

4. Utilisation d'une protéine EPI et d'héparine pour la préparation d'un médicament pour la prophylaxie ou le traitement de troubles de la coagulation ou du cancer.

5. Utilisation selon la revendication 4, dans laquelle la protéine EPI est liée à l'héparine.

6. Utilisation selon la revendication 4, dans laquelle la protéine EPI et l'héparine sont présentées dans des récipients séparés sous une forme adaptée à l'administration conjointe pratiquement simultanée ou successive de la protéine EPI et de l'héparine.
